(19)

**Europäisches Patentamt**
**European Patent Office**
**Office européen des brevets**

(11) **EP 2 034 921 B1**

(12) **EUROPEAN PATENT SPECIFICATION**

(45) Date of publication and mention
of the grant of the patent:
**10.10.2018 Bulletin 2018/41**

(21) Application number: **07719992.5**

(22) Date of filing: **19.06.2007**

(51) Int Cl.:
**A61B 34/00** *(2016.01)*   **A61B 90/00** *(2016.01)*

(86) International application number:
**PCT/CA2007/001076**

(87) International publication number:
**WO 2007/147232 (27.12.2007 Gazette 2007/52)**

(54) **APPARATUS FOR GUIDING A MEDICAL TOOL**

GERÄT ZUR FÜHRUNG EINES MEDIZINISCHEN WERKZEUGS

APPAREIL DE GUIDAGE D'UN OUTIL MÉDICAL

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.06.2006 US 814539 P**

(43) Date of publication of application:
**18.03.2009 Bulletin 2009/12**

(73) Proprietor: **Robarts Research Institute**
**London, ON N6A 5K8 (CA)**

(72) Inventors:
• **BAX, Jeffrey**
  **London, Ontario N6B 1X2 (CA)**
• **COOL, Derek**
  **London, Ontario N6G 5H9 (CA)**

• **GARDI, Lori Anne**
  **London, Ontario N5X 1J5 (CA)**
• **FENSTER, Aaron**
  **London, Ontario N6G 4N9 (CA)**

(74) Representative: **Faulkner, Thomas John et al**
**Cleveland**
**10 Fetter Lane**
**London EC4A 1BR (GB)**

(56) References cited:
**CA-A1- 2 552 589      US-A- 4 669 483**
**US-A- 4 723 544       US-A- 5 091 733**
**US-A- 5 257 998       US-A- 5 397 323**
**US-A- 5 817 084       US-A1- 2006 074 406**
**US-A1- 2004 193 777**

**Description**

**Field of the Invention**

The present invention relates generally to medical devices and, more particularly, to an apparatus for guiding a medical tool.

**Background of the Invention**

Apparatus for guiding medical tools have been shown to be of valuable assistance in various medical procedures, for example, manipulation of surgical tools, manipulation of cameras or sensors, biopsy, etc. An apparatus for guiding a medical tool usually also improves reproducibility compared to free-hand medical procedures, for example, surgical or biopsy procedures.

These apparatus typically have one or more degrees of freedom and may be manually driven in that the one or more degrees of freedom may be equipped with a brake with motive force being provided by a human practitioner, or may be automated in that at least one degree of freedom is driven by a computer controlled actuator.

A medical tool often needs to be oriented about a point in, on, or in proximity to a patient's body. However, having the main body of an apparatus that supports the tool located too proximal to the patient's body may be disadvantageous, since the supporting apparatus may, for example, interfere with the view of or access to the patient by the practitioner. An apparatus which can orient a tool about a remote fulcrum or remote center of motion can avoid such disadvantages.

The use of an apparatus that orients a tool about a remote center of motion is known in robotics as described, for example, in U.S. Patent Nos. 5,397,323, 5,515,478, 5,630,431, 5,817,084, 5,907,664, 6,047,610, 6,246,200, and 7,021,173. U.S. Patent No. 5,397,323 to Taylor et al. discloses the remote center of motion principle in surgical robots with a first axis of rotation pointing into the remote center of motion, and a second axis materialized by a parallelogram mechanism implemented by two coupled parallel linkages of rigid bars and cylindrical joints. The two axes of the remote center of motion are orthogonal, and the mechanism operated around an upright initial (zero) direction.

Unfortunately, the parallelogram structure of Taylor et al. and other conventional parallelogram mechanisms is bulky, making it difficult to position with respect to a patient's body and in some cases forcing a patient to assume an uncomfortable or unconventional position. Therefore, there is a need for an alternative apparatus for guiding medical tools. US 5257998 describes a medical three dimensional locating apparatus. The apparatus comprises a first arm pivotally supported for turning about a first axis, a second arm pivotally supported on the first arm for turning about a second axis perpendicular to the first axis and an indicating unit held on the second arm. US 4723544 describes a hemispherical vectoring needle guide for discolysis. The device has a target structure and a positioning component on which a guide mount for a needle guide is attached which are supported by an adjustment mechanism attached to an operating table.

US-A-4669483 shows a lithotripsy system having locating and orienting means.

It is an object of an aspect of the present invention to provide a novel apparatus for guiding a medical tool.

**Summary of the Invention**

In an aspect, there is provided an apparatus for guiding a medical tool, comprising:

a medical tool;
at least one crank arm comprising at least a portion of a first hinged coupling for hinged coupling to a stabilizer;
at least one link arm comprising at least a portion of a second hinged coupling for hinged coupling to the crank arm at a location spaced from the first hinged coupling;
a tool holder for supporting a medical tool on the link arm at a location spaced from the first hinged coupling;
wherein the rotational axes of the first and second hinged couplings intersect to define a remote fulcrum;
wherein the apparatus comprises a shaft, for actuating longitudinal and rotational motion of the medical tool, wherein the medical tool comprises a combination of an ultrasound transducer, a biopsy needle and a needle guide for allowing collection of ultrasound images for us in guiding operation of the needle.

**Brief Description of the Drawings**

Embodiments will now be described, by way of example only, with reference to the attached Figures, wherein:

Figure 1 is a front perspective view of a 3-element guide apparatus for guiding a transrectal ultrasound (TRUS) probe and biopsy needle with 'n' representing linkage elements, and 'l' representing hinged coupling axes;

Figure 2 is a front perspective view of the 3-element guide apparatus of Figure 1 attached to a multi-jointed stabilizer, which in turn may be attached to an operating room bed or fixture with 'n' representing linkage elements, and 'i' representing hinged coupling axes;

Figure 3(a) and (b) illustrate a 5-element, and (c) a 7-element closed loop spherical linkage with additional linkage elements used to provide additional support for the TRUS probe; 'n' representing connection elements, and 'i' representing hinge joint axes;

Figure 4 is a schematic of 2 rotational motions within the 5-element guide apparatus of Figure 3(a);

Figure 5 is an illustration of the spherical coordinate reference frame used to define the forward kinematics between the primary alignment axis (base) and tertiary alignment axis (probe tip);

Figure 6(a) is an exploded isometric view of the differential gear train used to decouple the rotation and linear travel of the TRUS about the tertiary alignment axis; Figure 6(b) is an isometric view of the differential gear train; Figure 6(c) is a top cross-sectional view of the differential train illustrating how the central shaft is coupled to the base and outer ring;

Figure 7 is an illustration of the top view of the guide apparatus of Figure 1 laid open on a surface showing the layout of the arcuate arms, braking sub-assembly, and encoders;

Figure 8(a) is an illustration of a trans-rectal ultrasound (TRUS) transducer with an attached biopsy guides showing an 18-gauge biopsy needle constrained within the imaging plane of the 2D US beam; Figure 8(b) shows a schematic diagram of the TRUS transducer, biopsy needle and guide in the rectum during a prostate biopsy; Figure 8(c) is an illustration of a TRUS image of the prostate with a biopsy needle (arrow) in the inner gland;

Figure 9 shows steps of a 2D and 3D prostate segmentation algorithm; (a) the user initializes the algorithm by placing 4 points on the boundary to generate an initial contour; (b) deformable dynamic contour approach is used to refine the initial contour until it matches the prostate boundary; (c) the contour is propagated to adjacent 2D slices of the 3D TRUS image and refined; the process is repeated until the complete prostate is segmented as shown.

## Detailed Description of the Embodiments

A guide apparatus can be useful for guiding a medical tool in 3D space. A guide apparatus may comprise one or more rotational degrees of freedom and an adaptable cradle for coupling a medical tool. Using this guide apparatus, physicians can maneuver a medical tool to a desired 3D position and orientation.

The guide apparatus is capable of producing a remote fulcrum and can be configured to constrain movement of a medical tool relative to the remote fulcrum. The constrained movements produced by the guide apparatus are consistent with movements produced by a user during a conventional surgical procedure. When the instrument is manipulated manually, the guide apparatus will passively follow the user's movements while still maintaining orientation of a medical tool relative to a fixed remote fulcrum that may be positioned to coincide with a restricted entrance point of a patient's body, for example a rectum or any surgical incision. Since the guide apparatus constrains the orientation of a medical tool relative to and through a fixed point in space, a user's movements are reproduced at a scaled down rate (minimized through the remote fulcrum) that allows for a level of precision that was thought to only be possible with robotic assisted machines. This improves the ability of a user to accurately target a point of interest within a patient's body.

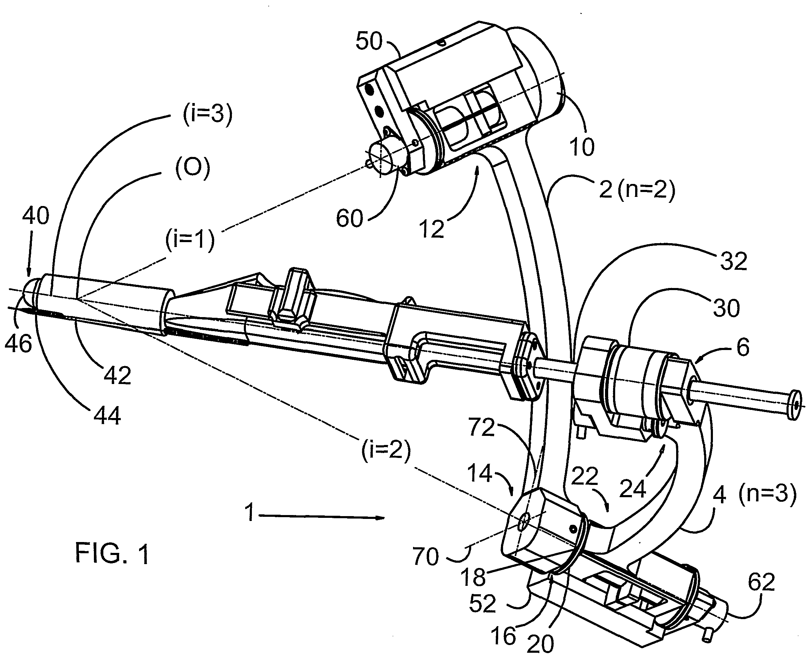

Figure 1 shows an example of a guide apparatus 1 that may be used for 3D orientation of a medical tool relative to and through a fixed point in space, a remote fulcrum. The guide apparatus comprises two linkage elements or arms, a crank 2 and a link 4. The crank 2 and the link 4 may be of any size, or shape that allows for the remote fulcrum 0.

The linkage elements may be hingedly coupled to form positioning elements. In Figure 1 the crank 2 and link 4 both have an arcuate structure having a central angle of about 45 degrees. The crank has a first end 12 and a second end 14. The link also has first and second ends 22, 24. When the guide apparatus is in use the first end 12 of the crank is hingedly coupled to a base or stabilizer. The first end 12 may comprise a full hinged coupling (not shown) that is attached to a member that is rigidly fixed to the base or stabilizer. Alternatively, the first end 12 may comprise a portion of a hinged coupling 10 with the remainder of the hinged coupling being provided by the base or stabilizer. The second end 14 of the crank forms a hinged coupling 16 with the first end 22 of the link. The second end 14 of the crank comprises a portion 18 of the hinged coupling 16, while the first end 22 of the link comprises the remaining portion 20 of the hinged coupling 16. The second end 24 of the link is coupled to a tool holder 6. The tool holder may be in the form of an adaptable cradle for securing a shaft 32 that may be used to actuate a medical tool 40.

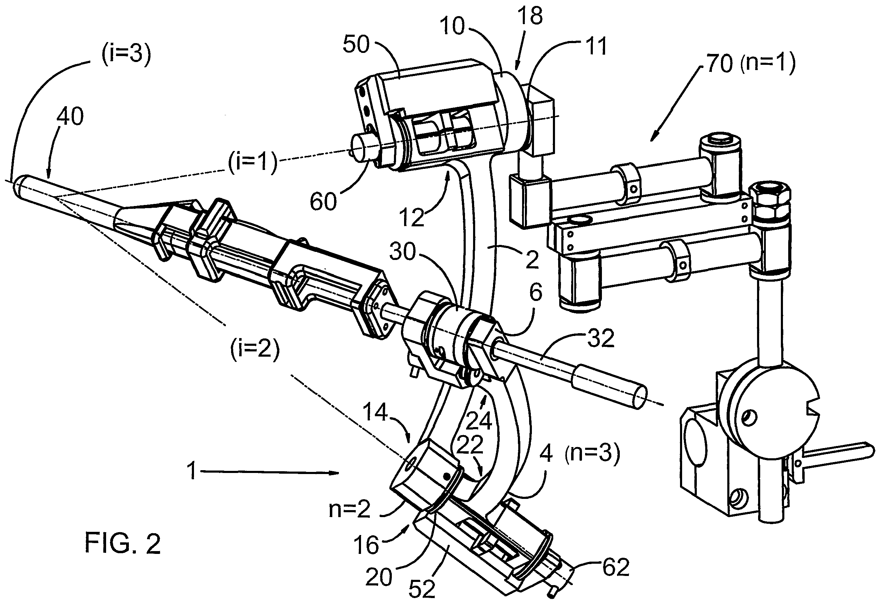

Figures 1 and 2 show a medical tool 40 and a shaft 32 for actuating the medical tool. The shaft may be used to actuate longitudinal and/or rotational or angular motion of medical tool 40 relative to the tertiary alignment axis; longitudinal or linear motion along the axis provides one degree of freedom, while rotational or angular motion about the axis provides another degree of freedom. The shaft 32 passes through a cylindrical joint provided by tool holder 6. The shaft 32 may be coupled directly to the medical tool 40, or may be coupled to a sleeve or any other convenient structure for receiving the medical tool 40. The medical tool 40 shown in Figure 1 is a combination of a transrectal ultrasound (TRUS) transducer 46, a biopsy needle 44 and a needle guide 42.

A remote fulcrum 0 produced by the guide apparatus 1 is shown in Figures 1 and 2. As shown in Figure 2 the remote fulcrum 0 is formed at an intersection of the rotational axis (i=1) of the first hinged coupling formed between the first end 12 of the crank and the base or stabilizer 70 and the rotational axis (i=2) of the second hinged coupling formed between the second end 14 of the crank and the first end 22 of the link. When the guide apparatus is in use and is coupled to the medical tool 40 the axis (i=3) of the medical tool 40 passes through the remote fulcrum. In certain examples, the axes of the medical tool 40 and its shaft actuator 32 are collinear and both pass through the remote fulcrum.

The guide apparatus may be equipped with further components as desired to aid in the orientation or tracking of a medical tool, for example, without limitation, brakes for locking a hinged coupling, encoders for measuring rotational angles of a hinged coupling, counterweights and/or spring balances to offset the mass of the system, computer controlled actuators for automating rotation of a hinged coupling, additional linkage arms or the use of linkage arms having an adjustable arcuate structure. Further components that may be incorporated into the guide apparatus will be apparent to the skilled person, and suitable combinations of optional components will also be apparent depending on the particular medical tool and the particular use of the guide apparatus.

One example of an optional component that may be included in a guide apparatus is a rotational encoder. As seen in Figures 1 and 2, a first rotational encoder 60 that may be mounted to the first end 12 of the crank 2, while a second rotational encoder 62 may be mounted to the first end 22 of the link 4.

As another example of an optional component, counterweight 52 is mounted to the link arm to offset the mass of a medical tool and associated hardware supporting it; while counterweight 50 is mounted to the crank arm to offset the mass of the crank arm, counterweight 52, and the link arm. The counterweights may be replaced or used in conjunction with a spring balance to offset the mass of the system.

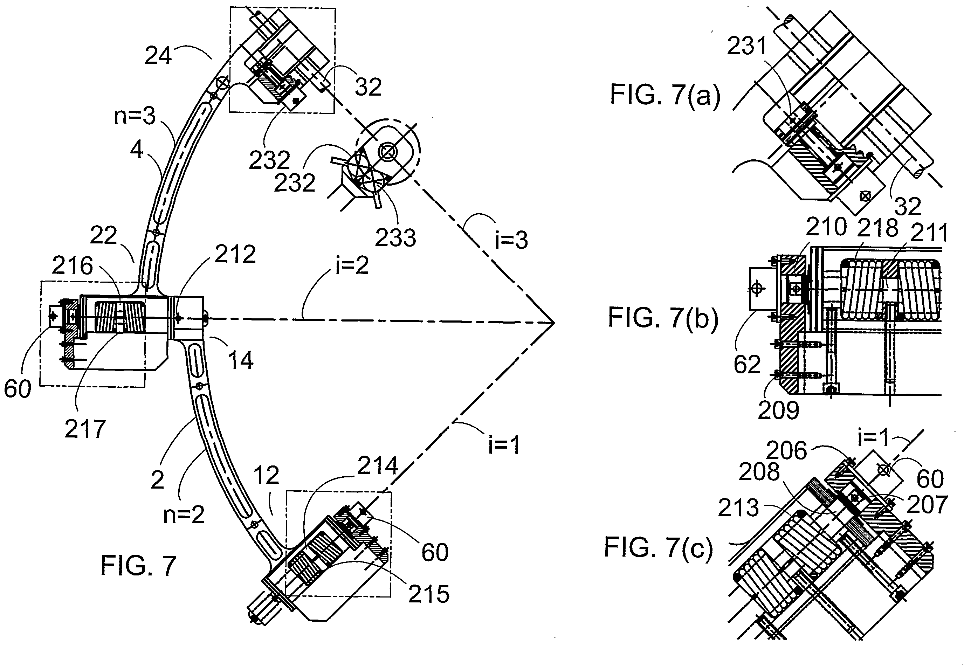

As yet another example of an optional component, a braking mechanism may be mounted within the crank and/or the link to inhibit motion of linkage elements relative to each other. In one example, a spring clutch may be mounted within the first end 12 of the crank arm to prevent or inhibit motion of the crank relative to the stabilizer or base fixture. The spring clutch (shown in Figure 7) may be comprised of two brake pads, in which at least one of the brake pads is affixed to the first end 12 of the crank, and at least one torsion spring is wrapped around the pair of brake pads.

As still another example of an optional component, a guide apparatus may be equipped with motors (not shown), for example servo motors that may be controlled by a computer to automate the motion of various linkage elements. In a particular example, each hinged coupling independently may be controlled by a servo motor.

As a further example of an optional component, a guide apparatus with an adjustable remote fulcrum may be produced by incorporating linkage arms having an adjustable arcuate structure (not shown). To make the remote fulcrum adjustable, an additional two hinged couplings can be integrated into the guide apparatus shown in Figure 1. The first additional hinged coupling can be located between 12 and 14 of the first crank with its axis of rotation being parallel to the axis 70. By adjusting the angle between 12 and 14, the point of intersection between i=1 and i=2 can be changed. In order to maintain the remote fulcrum, a second additional hinged coupling on link 4, between 22 and 24 with its axis of rotation parallel to the axis 72, can be used to adjust the intersection point between i=2 and i=3 to coincide with the previous adjustment. This optional component is particularly useful to adjust the remote fulcrum for different medical tools and/or different uses, or to account for manufacturing tolerances in the device. Typically, the remote fulcrum of the guide apparatus would be adjusted prior to a surgical procedure, set in place, and then maintained in a fixed position throughout the procedure. A planar coupling can be used in place of the first additional hinged coupling between 12 and 14 if the planar connection is parallel to the plane formed by the axis i=2 and 72. A planar coupling can be used in place of the second additional hinged coupling between 22 and 24 if the planar connection is parallel to the plane formed by the axis i=2 and 70.

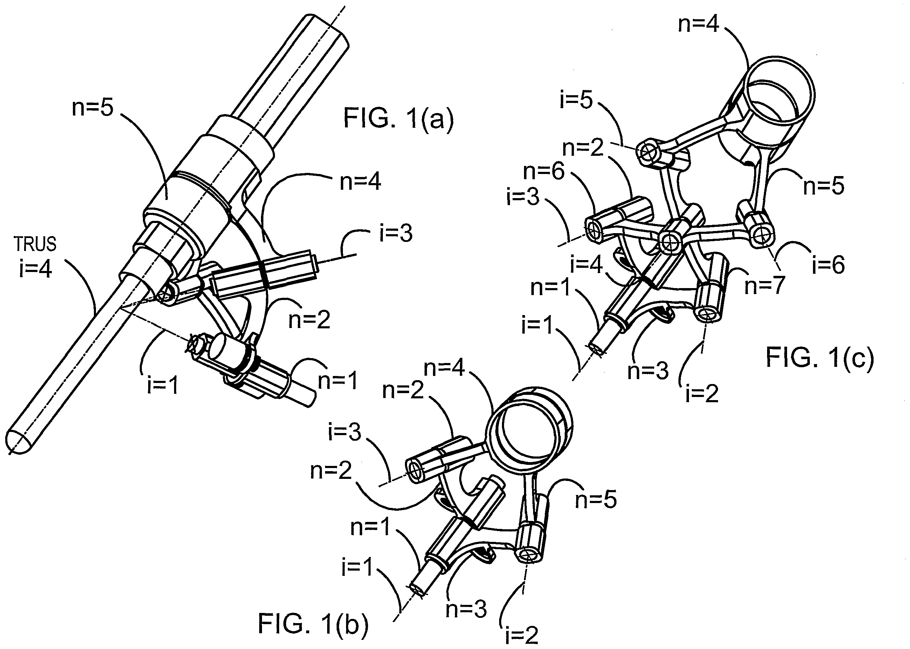

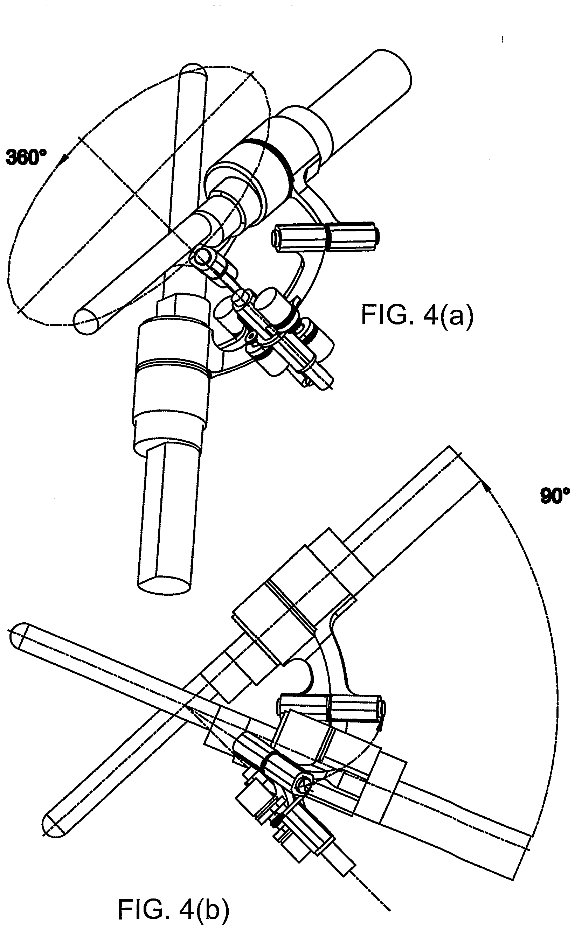

An even further example of an optional component are further linkage elements, for example a second crank arm and a second link arm. While the guide apparatus has so far been described as comprising two linkage elements, Figures 3 and 4 show that the guide apparatus can be configured two comprise further linkage elements and be converted from an open-loop spherical chain to a closed-loop spherical chain. For example, to reduce inertia effects from the use of two arcuate linkage elements in an open-loop spherical chain, 2 or 4 additional linkage arms (Fig. 3) may be integrated into the design and form a closed-loop spherical chain. The closed chain design can dampen the inertia effects present in the open chain design illustrated in (Fig. 1). This would be useful for applications where improved sensitivity is required for finer adjustments (e.g. small animal interventions), or the guide apparatus is supporting relatively large payloads (e.g. the addition of motors to automate the motion of the guide apparatus under the control of a computer). Additional linkage elements (Fig. 3) may also be useful for supporting a number of different medical tools of varying sizes and weights. The closed-loop spherical chain is more capable of supporting unbalanced loads from the additional support provided by the additional linkage illustrated in (3b) and (3c). As can be seen in Figure 3a-c, the tool holder can be constructed as two separate portions that are independently linked to the link arms. The ring portions may be free of each other and may be aligned by a shaft passing through the rings. The ring portions may also be rotatably coupled to each other.

When the guide apparatus is manipulated manually, the closed kinematics frame will follow the user's hand movements with minimal resistance. Accordingly, any number of different paths of motion may be achieved by the guide apparatus. Two paths of motion that are intuitive to most user's are illustrated in Figures (4a) and (4b). As illustrated in Figure (4a), the apparatus can revolve about the base alignment axis of a hinged coupling between the first end of the crank and the base or stabilizer. This rotation becomes more apparent as the angle between the medical tool axis and the base alignment axis increases. There is also a natural tendency for many user's to change the angle between the medical tool axis and the base alignment axis as this produces a side-to-side motion of the medical tool about the remote fulcrum point of the guide apparatus. As shown in Figure 4b, as each of the cranks rotates away from one another, the opposing inertial forces, which are generated within the linkage, will direct the medical tool along a path which is perpendicular to the path of motion previously described for Figure 4a. Therefore, the additional two linkage elements are useful as they reduce the effect of inertial influence in comparison to a corresponding open-loop chain design. User's are able to recreate the intuitive paths of motion shown in Figures 4a and 4b with reduced veer or drag due to inertial forces of a heavy medical tool.

Various configurations of linkage arms or hinged coupling are readily available to the skilled person. For example, the crank arm 2 and the link arm 4 may be of any size, or shape that allows for a configuration of the guide apparatus that produces a remote fulcrum 0. The crank and the link may be of equal length, the crank may be longer than the link, or the crank may be shorter than the link. The crank and the link may be the same or different in terms of rigidity or flexibility. The crank and the link will typically be arcuate, and the crank and the link may be the same or different in terms of arcuate structure. The arcuate structure may have any suitable central angle for maintaining a remote fulcrum. For example, an arcuate crank or an arcuate link may each independently have a central angle of about 10, 20, 30, 40, 50, 60, 70, 80, 90, 100, 120, 130, 140, 150, 160, 170, 180, 190, or 200 degrees, or any suitable angle therebetween. Typically, the central angle will be less than 360, 330, 300, 270, 240, 210, 180, 150, 120, 90, 60, or 30 degrees, or less than any angle therebetween.

Hinged couplings do not need to be placed at the end of linkage elements. For example, the first end of the crank arm may extend beyond the first hinged coupling, As another example, the second end of the crank arm and/or the first end of the link arm may extend beyond the second hinged coupling. The link arm is coupled to the crank arm at a second hinged coupling sufficiently spaced from the first hinged coupling to achieve two positioning elements and such that their rotational axes can define a remote fulcrum.

Still further optional features will be apparent to the skilled person.

While the guide apparatus 1 shown in Figures 1 and 2 has so far been described in terms of structural features, a guide apparatus may also be described in terms of its axial components and planes defined by the axial components. The guide apparatus typically comprises a primary (or base) alignment axis (i=1); a secondary alignment axis (i=2) that intersects the base alignment axis at a remote fulcrum point 0 and at a fixed angle to the base alignment axis, defining a first plane that represents a first positioning element; a tertiary alignment axis (i=3) which intersects the base and secondary alignment axes forming a fixed angle between the secondary and tertiary axes, and defining a second plane that represents a second positioning element; and, the first, and second positioning elements are separately adjustable in order to provide a pre-determined and/or angular relationship between the base and tertiary alignment axes. The first, and second positioning elements are separately adjustable to allow for 3D orientation of the tertiary alignment axis through the remote fulcrum.

As seen in Figures 1 and 2, the crank 2 of the guide apparatus is parallel to the first plane defined by the intersection of a base alignment axis (i=1) and a secondary alignment axis (i=2), while the link 4 is parallel to the second plane defined by the intersection a secondary alignment axis (i=2) and a tertiary alignment axis (i=3).

The first and/or second positioning elements may be manually, automatically or both manually and automatically adjustable. The first and second positioning elements provide for adjustment of the distance between the primary alignment axis (i=1) and the tertiary alignment axis (i=3) by adjusting the polar position of the first and second positioning elements. As seen in Figures 1 and 2, adjustment of the angular displacement between the primary and tertiary alignment axis may comprise a link arm having a first end 22 hingedly coupled to a second end 14 of a crank arm where its first end 12 may be hingedly coupled to a grounded fixture or stabilizer 70 shown in Figure 2. The second end 24 of the link arm may comprise a cylindrical joint to which medical tool(s) 40 may be coupled. Typically, the base, secondary and tertiary axes of the revolute hinged couplings and cylindrical joints of the crank and link converge to a remote fulcrum point 0, thereby forming an open-loop spherical chain.

## Kinematics Equations of Motion

The guide apparatus may be considered as a coordinated spherical linkage assembly, which comprises two hinged couplings and three linkage elements. The axis of each hinged coupling converges to a common point to produce a remote fulcrum. The linkage assembly is a compound spherical joint with two degrees of freedom (DOF), as defined by the Kutzbach criterion:

$$DoF(n,j) = l(n-1) + \sum_{i=1}^{j}(p_i - 1)(f_i - 1) \qquad [1]$$

where:

'n' represents the total number of connected elements and 'j' is total number of lower pair joints in the mechanism. For a single joint, 'i', the relative mobility of the joint and the number of elements connected to it are given by '$f_i$' and '$p_i$', respectively. The mobility of each linkage element relative to each other is quantified by 'l'.

Equation 1 is useful for analyzing a complex linkage to quantify its mobility and/or to determine the degrees of freedom provided by the linkage.

The first hinged coupling defines the reference axis of the coordinate system and is fixed to the multi-jointed stabilizer that may be attached to an exam room bed (or fixture). Because each linkage element is constrained to pivot about a common point (ie., the remote fulcrum), the mobility of one linkage element, I, is constrained to three degrees of rotation. The angular size and length of each element in the linkage assembly defines the size and shape of the operating envelope of the kinematics frame.

The spherical linkage assembly supports a medical tool and its associated supporting elements through a tool holder so that the longitudinal axis of the medical tool is collinear with the tertiary alignment axis (i=3). The angular position of the axis of the medical tool, relative to the base alignment axis, is determined by measuring the angle between the base and secondary alignment axes. As shown in Figure 1, shaft 32 with a sleeve may be coupled to a cylindrical joint provided by the tool holder, with the sleeve adapted to receive the medical tool 40. The cylindrical joint allows the shaft, sleeve and medical tool, to pivot and slide freely along the axis of the tertiary alignment axis, providing an additional two DOF as the probe penetration and relative rotational angle to the supporting frame are defined.

The following equations represent the forward kinematics equations of motion for the open-loop linkage:

$$\tan\tfrac{1}{2}(\theta + c) = \cos\tfrac{1}{2}(\psi - \tfrac{\pi}{2})\sec\tfrac{1}{2}(\psi + \tfrac{\pi}{2})\cot\tfrac{1}{2}\zeta \qquad [2]$$

$$\tan\tfrac{1}{2}(\theta - c) = \sin\tfrac{1}{2}(\psi - \tfrac{\pi}{2})\csc\tfrac{1}{2}(\psi + \tfrac{\pi}{2})\cot\tfrac{1}{2}\zeta \qquad [3]$$

$$\tan\tfrac{1}{2}\varphi = \tan\tfrac{1}{2}(\psi - \tfrac{\pi}{2})\sin(\theta + c)\csc(\theta - c) \qquad [4]$$

$$\tan\tfrac{1}{2}\psi = \cos\xi \qquad [5]$$

$$\tan\tfrac{1}{2}(\gamma + \xi) = \cos\tfrac{1}{2}(\psi - \tfrac{\pi}{4})\sec\tfrac{1}{2}(\psi + \tfrac{\pi}{4})\cot\tfrac{1}{2}\xi \qquad [6]$$

$$\tan\tfrac{1}{2}(\gamma - \xi) = \sin\tfrac{1}{2}(\psi - \tfrac{\pi}{4})\csc\tfrac{1}{2}(\psi + \tfrac{\pi}{4})\cot\tfrac{1}{2}\xi \qquad [7];$$

or

Equation 2 to 5 and

$$\cot\tfrac{1}{2}\xi = 1/\sqrt{2}\tan\gamma \qquad [7a].$$

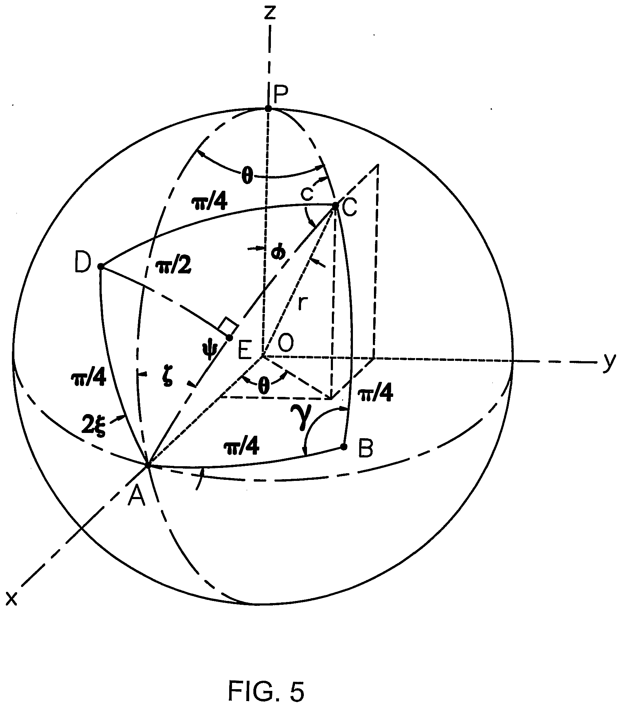

Equations 2, 3 and 4 were derived by applying the Napier analogies to spherical triangle APC (Fig. 5), and Equation 5 was determined by solving the right spherical triangle ABE. Applying the Napier analogies to spherical triangle ABC, gives Equations 6 and 7. Equation 7a is derived by solving the right spherical triangle ABE.

Equations 2-7 are useful to calculate the orientation of the medical tool in 3D space relative to the remote fulcrum based on encoder positions in the open-loop chain design. Alternatively, replacing Equation 6 and 7 with 7a, Equations 2-5 and 7a are useful to calculate the orientation of the medical tool in 3D space relative to the remote fulcrum based on encoder positions in the open-loop chain design. For, corresponding calculations for the closed-loop chain design can be performed using Equations 2 to 5.

The position vector, r defining the 3D position of the medical tool relative to its fulcrum is defined as:

$$\bar{r} = \begin{bmatrix} x \\ y \\ z \end{bmatrix} = \begin{bmatrix} r\cos\theta\sin\varphi \\ r\sin\theta\sin\varphi \\ r\cos\varphi \end{bmatrix} \qquad \text{(see Figure 5)} \qquad [8].$$

Equation 8 is useful for coordinate transformation from a spherical coordinate system (which references angles as with Equations 2-5 or Equations 2-7 or Equations 2-5 and 7a) into a cartesian coordinate system (x,y,z) with the origin being a remote fulcrum 0.

The encoders 60, 62 mounted to the hinged couplings (Fig. 2, i = 1, 2) are used to measure the angles $(\zeta+\xi)$ and $\gamma$, respectively.

In order to uniquely define the orientations of the medical tool about the remote fulcrum, as defined by the vector **r** (fig. 5), information about any two of the three possible angles (gamma (angle ABC), xi (angle CAB), and zeta (angle PAC) designated as $\gamma$, $\xi$, and $\zeta$, respectively) measured by the encoders at the hinged couplings is needed to solve the forward kinematics equations.

In one example, the position of each arm (AB and BC in figure 5) in the linkage is determined by measuring the spherical angles at each of the hinged couplings A and B, respectively. The encoder mounted at 'A' would measure the angle $(\xi+\zeta)$, and the encoder mounted at the second hinged coupling 'B' would be used to measure the angle between the two arms $(\gamma)$. Equation 6 and 7 or Equation 7a can be used to decouple the values for $(\xi)$ and $(\zeta)$, required to solve the Equations 2-5.

In an alternate example, where an additional two arms are incorporated to produce a closed-loop chain design (fig. 3), the encoder mounted at point B, can also be mounted at points D or A. If the second encoder were mounted at coupling 'D', the analysis described in the previous paragraph would be used since the encoder provides the same information. However, if both of the encoders were mounted at point 'A', each encoder would be used to measure the angle of rotation of link AD and AB about the x-axis (fig. 5). The encoder mounted on arm AD would measure $(\zeta-\xi)$, and the encoder mounted on arm AB would measure $(\xi+\zeta)$. This would provide enough information to solve Equations 2-5 without the need for Equations 6 and 7 or Equation 7a. Since the encoders can be mounted in different configurations, Equations 2-5 can be used to optimize the encoder placement for a particular application. This is because the encoder sensitivity to movement is different for each of the cases described above.

Degrees of freedom of the guide apparatus may be provided by hinged coupling of linkage elements. Additional degrees of freedom may be provided depending on the medical tool and its associated hardware and actuator. For example, Figure 1 shows a medical tool 40 and a shaft 32 for actuating the medical tool, with the medical tool axis and the shaft axis being collinear with the tertiary alignment axis (i=3). The shaft may be used to actuate longitudinal and/or rotational or angular motion of medical tool 40 relative to the tertiary alignment axis; longitudinal or linear motion along the axis provides one degree of freedom, while rotational or angular motion about the axis provides another degree of freedom. The shaft may be equipped with a lockable collar to prevent linear motion of a medical tool during a procedure. The shaft 32 passes through a cylindrical joint provided by tool holder 6 and is coupled to a differential gear train 30 that is housed within tool holder 6. The differential gear train may be used to decouple degrees of freedom, for example linear and angular motion about an axis. Furthermore, the differential gear train may be equipped with or coupled to encoders to measure each decoupled degree of freedom.

As illustrated in Figure 1, a differential gear mechanism 30 housed within tool holder 6, mechanically decouples two degrees-of-freedom provided by the shaft and its coupling to the tool holder. These degree of freedoms represent the linear and the angular orientations, respectively of the shaft 32 and its associated medical tool about a longitudinal axis.

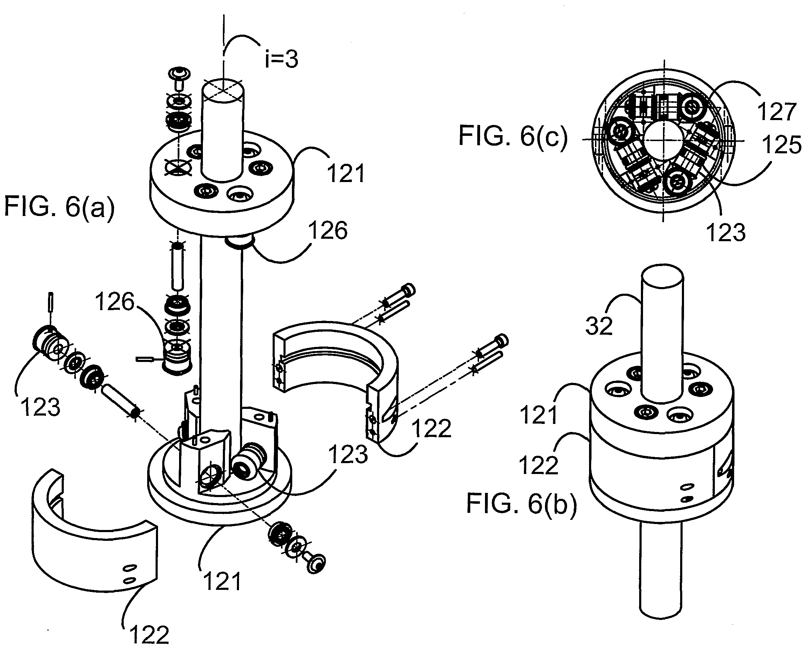

Referring to Figure 6, an example of a differential gear train is illustrated. The differential gear train comprises of three basic components:

- Base Drum (121)
- Planetary Gear Train (123 and 126)
- Outer Ring (122).

Referring to Figures 6 and 7, the angular and linear displacement of a medical tool about the tertiary alignment axis is measured using two rotary (rotational) encoders (Figure 7: 232, 233), by measuring the angular displacement of the base drum (Figure 6:121) and outer (122) rings respectively. Three miter gears 123, whose axis are perpendicular to the tertiary alignment axis, are connected to the shaft 32 by a friction wheel 125. In an alternate embodiment, a spur gear meshing with a rack embedded within a splined shaft would be used in place of the friction wheel 125. Meshing with the inner gears 123, are a set of three matching miter gears 126 pivotally attached to the inner ring 121, and axis of rotation parallel to the tertiary alignment axis, transfers the linear displacement of the shaft to a rotational movement that is aligned with the longitudinal axis of the shaft. The three miter gears 126 attached to the base drum 121, engages with the inner diameter of the outer ring 122 by means of a friction wheel 127. In an alternate embodiment, a spur gear meshing with an internal gear mounted to the inner ring would be used in place of the friction wheel 127.

## Rotational Motion of the Differential Gear Mechanism

The base drum, which has an outer diameter (D = 1.75 inches), is mechanically coupled to the shaft 32 (see Figure 7) and to one of the two encoders having a friction-wheel (see 231 in Figure 7, which has a diameter, $d_{encoder}$ = 1.0 inches). As shown in Equation 9, the ratio of the drum diameter, D, to the friction wheel diameter of the encoder, $d_{encoder}$, determines the error reduction ratio between the encoder and the positional accuracy of the medical tool ($\mu_{reduction}$).

$$\mu_{reduction} = \frac{D}{d_{encoder}} \qquad [9]$$

$$\mu_{reduction} = 1.8$$

As shown in Equation [10], the ratio of the encoder accuracy, $\delta_{encoder}$, (Renishaw 2006) to the error reduction ratio, $\mu_{reduction}$, defines the accuracy of the rotational motion for the shaft 32 ($\delta_{shaft}$).

$$\delta_{shaft} = \frac{\delta_{encoder}}{\mu_{reduction}} \qquad [10]$$

$$\delta_{shaft} = \pm 0.29°$$

## Linear Motion Using a Differential Gear Mechanism

The planetary gear train 123,126, which comprises three pairs of miter gears, converts the longitudinal or linear movement of the shaft (i.e. penetration of the shaft along its axis into a subject's body) to a rotational motion of the outer ring (see 122 in Figure 6).

As the shaft 32 is displaced 1.0 inches along the longitudinal direction, the 1:1 ratio of the miter gears (Berg M72N-72-S) produces a displacement of 1.0 inches along the inner diameter of the outer ring (see Item 122 in Figure 6). As shown in Equation [11], this movement results in an angular displacement of the outer ring:

$$\Delta D_{angular} = \frac{1.0''}{\pi \cdot d} \cdot 360° \quad , \text{ where } d = 1.387 \text{ inches} \qquad [11]$$

$$\Delta D_{angular} = 82.618°$$

Because the friction wheel of the encoder, ($d_{encoder}$ = 1.0 inches), is coupled to the outside diameter of the outer ring (D = 1.75), the accuracy of the encoder (Renishaw 2006) is minimized (see Equation [9]). Equation [9] is combined with the results of Equation [11] in order to obtain the encoder sensitivity for the penetration of the shaft and its associated medical tool, $\delta_{penetration}$:

$$\delta_{penetration} = \frac{\pm 0.5°}{\Delta D_{angular} \cdot \mu_{reduction}} \cdot 1.0'' \qquad [12]$$

As mentioned above, the guide apparatus may be equipped with optional components as desired to aid in the orientation or tracking of a medical tool, for example, without limitation, brakes for locking a hinged coupling, encoders for measuring rotational angles of a hinged coupling, counterweights and/or spring balances to offset the mass of the system, computer controlled actuators for automating rotation of a hinged coupling, or additional linkage arms. Further components that may be incorporated into the guide apparatus will be apparent to the skilled person, and suitable combinations of optional components will also be apparent depending on the particular medical tool and the particular use of the guide apparatus.

Particular examples of encoders, counterweights and braking mechanisms are now described.

Referring to Figure 7, to determine the spatial orientation of the tertiary alignment axis to the base alignment axis, two rotational encoders 60,62 mounted to a first end 12 of the crank 2, and a first end 22 of the link 4 are used to measure the polar rotation of the crank arm relative to the base fixture, and the relative angles between the crank arm and the link arm. To measure the angle between the base fixture and the crank arm, a rotational encoder 60, mounted to a first end 12 of the crank by fasteners 206 measures the relative angular orientation of the encoder magnet 207 fixed to the shaft 208, which is in turn is rigidly mounted to a fixture or stabilizer. To measure the angle between the crank arm and the link arm, an angular encoder 62 mounted to a first end 22 of the link by fasteners 209 measures the relative angular orientation of the encoder magnet 210 fixed to the shaft 211, which is in turn is rigidly mounted to the second end 14 of the crank by pin 212.

Referring to Fig. 1, to dynamically balance the guide apparatus, counterweights may be affixed to the crank arm and/or the link arm. The counter weight 52 mounted to the first end 22 of the link is in place to offset the mass of a medical tool and associated hardware supporting it; while counterweight 50 mounted to the first end 12 of the crank is in place to offset the mass of the crank arm, counterweight 52, and the link arm. In an alternate embodiment, the counterweights may be replaced or used in conjunction with a spring balance to offset the mass of the system.

Referring to Fig. 7, a first end 12 of the crank arm comprises a spring clutch to prevent movement of the crank about the attached base fixture or stabilizer. The spring clutch comprised of two brake pads (214 and 215) in which at least one of the brake pads is affixed to the first end 12 of the crank, and at least one torsion spring 213 is wrapped around the pair of brake pads. When the torsion spring is in its relaxed state, the inner diameter of the spring must be smaller than the outer diameter of the brake pads. When the torsion spring(s) are mounted onto the brake pads, the force of the spring causes the pads to collapse onto the shaft, which in turn is rigidly fixed to the stabilizer or fixture. The frictional force generated by this clamping action prevents the crank arm from rotating about the primary alignment axis.

Referring again to Figure 7, the second positioning element 250 includes a spring clutch integrated into a first end 22 of the link arm to prevent movement of the link arm about the secondary alignment axis. The spring clutch comprised of two brake pads (216 and 217) in which at least one of the brake pads is affixed to the first end 22 of the link arm, and at least one torsion spring 218 wrapped around the pair of brake pads. When the torsion spring is in its relaxed state, the inner diameter of the spring must be smaller than the outer diameter of the brake pads. When the torsion spring(s) are mounted onto the brake pads, the force of the spring causes the pads to collapse onto the shaft, which in turn is pinned to the first positioning means. The frictional force generated by this clamping action prevents the link arm from rotating about the secondary alignment axis.

As will be recognized by the skilled person, the guide apparatus may be used for different medical applications using a variety of medical tools. In one particular example, a guide apparatus may be used as a 3D mechanically tracked transrectal ultrasound (TRUS) prostate biopsy system.

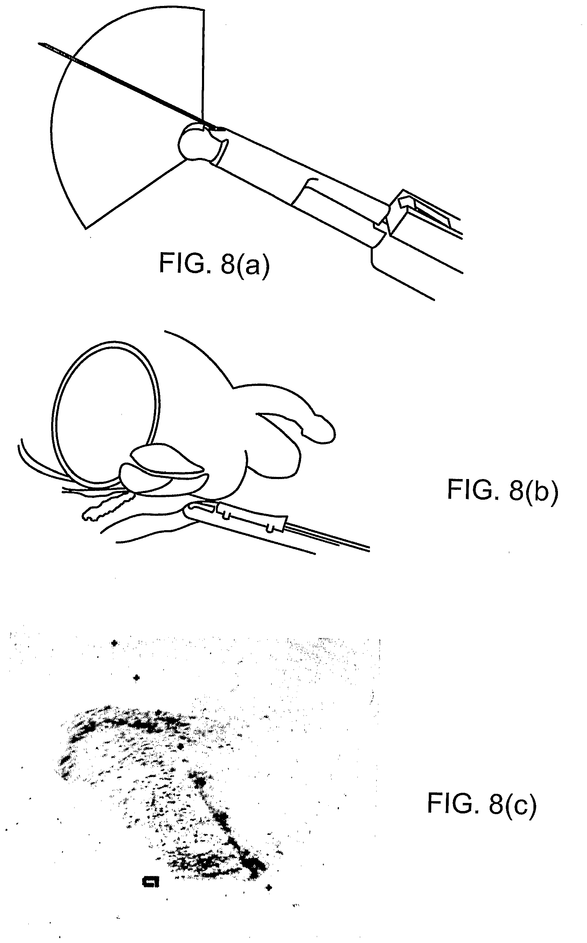

Definitive diagnoses of prostate cancer are typically determined from the histological assessments of tissue samples drawn from the prostate during biopsy procedures. Most biopsies are performed by a physician using a transrectal ultrasound probe (Fig. 8a, b) which uses a needle guide attached to the probe in order to constrain an 18 gauge needle so that it is always visible in the 2D US image (Fig. 8c). Each biopsy core is identified separately as to its location. As a result, so the pathologists can report the extent and the grade of the cancer. Depending on the pathological results of a biopsy procedure, urologists must either avoid the previously targeted biopsy sites or target those locations directly. Therefore, it is important to know exactly where the initial sample was taken in order to target more relevant tissue if the pathologist requests a repeat biopsy.

Currently, physicians are limited to using 2D transrectal ultrasound for guiding a biopsy needle into the prostate. Since 2D ultrasound images do not provide any spatial information about the location of the biopsy sample, it is difficult for physicians to plan repeat biopsy procedures.

A guide apparatus forms part of an effective mechanical 3D biopsy system that addresses the limitations of current 2D biopsy procedures, and minimizes the cost and retraining the physician must acquire. The biopsy system consists of a 4 degree-of-freedom guide aparatus comprising an adaptable cradle that supports a commercially available trans-rectal ultrasound transducer. Using this apparatus, physicians can maneuver an ultrasound transducer while a tracking system records the 3D position and orientation of the biopsy needle in real-time.

This approach involves the use of a device composed of two mechanisms (Figs. 1, 2):

    a. an articulated multi-jointed stabilizer (Fig. 2), and
    b. the guide apparatus shown in Figure 1 having a TRUS transducer 46, a needle guide 42, and biopsy needle 44.

The end-firing TRUS transducer (with the biopsy needle guide in place (42, Fig. 1) is mounted to the guide apparatus in a manner where the TRUS transducer is actuated by shaft 32 for rotational and linear movement along the longitudinal axis of the TRUS transducer. This will allow the physician to insert the TRUS transducer through the restricted opening of the patient's rectum and to rotate it in order to acquire a 3D image of the prostate. In certain examples, the multi-jointed stabilizer does not contain angle sensing encoders. However, the guide apparatus comprises angle sensing encoders mounted to each joint in order to measure the angle between the arms as well as the rotational and longitudinal motion of the shaft 32 and its associated medical tool 40, in this case the TRUS transducer 46, a needle guide 42, and biopsy needle 44. Information from the encoders is transmitted to a computer for further processing. This arrangement will allow the computer to determine not only the relative position of the transducer but also the needle relative to remote fulcrum 0.

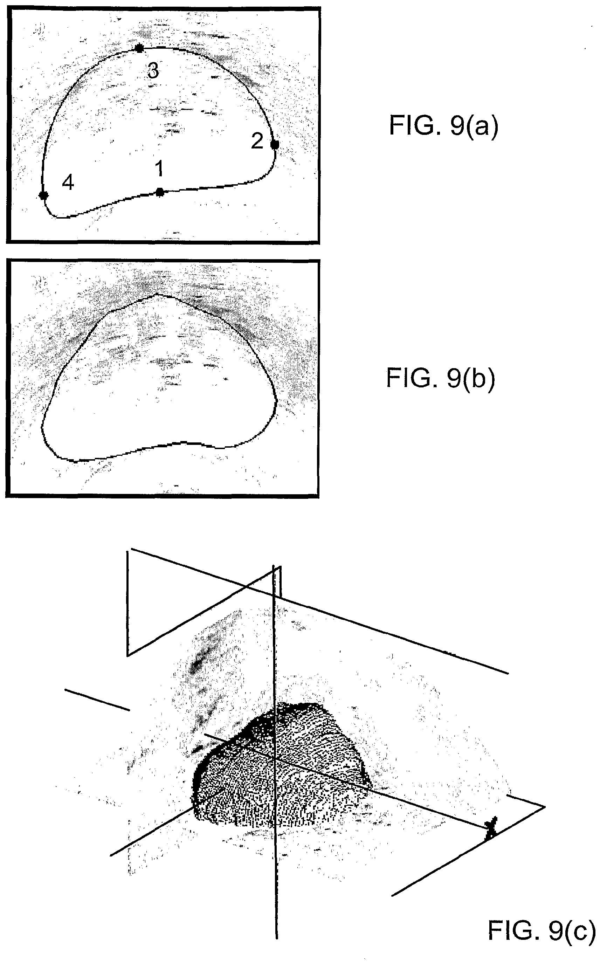

In use, the TRUS transducer is mounted into the guide apparatus such that the tip of the probe is initially set to the remote fulcrum point of the guide apparatus. The multi-jointed stabilizer is unlocked and the physician manipulates the transducer (the fulcrum of the guide apparatus), to the patients rectal sphincter. The stabilizer mechanism is then locked and the probe is inserted into the patient's rectum. The physician (or a motor) rotates the probe about its longitudinal axis to acquire a 3D TRUS image of the prostate. The prostate is then segmented using a manual semi-automated segmentation algorithm. An example of prostate segmentation is shown in Figure 9. Further information (e.g, functional, anatomical or probability image), if available, is registered to the 3D TRUS image and displayed as an overlay on the computer screen (Fig. 9). After the target in the 3D TRUS image is chosen using the US image as a guide, one or more linkage elements of the guide apparatus are then unlocked using a separate braking system then the one used to lock the stabilizer. The transducer is then free to allow the physician to move it to a new location while the TRUS probe and needle position is tracked by the encoders and associated software. At the same time, the needle trajectory is continuously displayed as a graphic overlay in the 3D TRUS image. When the needle path intersects the chosen target, the linkage elements of the guide apparatus are locked in place and a biopsy is performed in real time using 2D US guidance. The biopsy location is then recorded in 3D from the tracker orientation, and the system is ready for the next biopsy. After the needle is withdrawn, a 3D image may be obtained to determine if there is any movement or swelling of the prostate.

Biopsies are typically performed with a thin, 18-guage needle mounted on a spring-loaded gun connected to the ultrasound ("US") probe, forcing the needle to stay in the imaging plane so that it is always visible in the US image. The location of each core is registered, so that the pathologist can report the extent and grade of the cancer. This is especially important if the histological result is equivocal and the pathologist requests a repeat biopsy. It is, therefore, important to know from what exact location the = sample was obtained in order to target more relevant tissue if a repeat biopsy is performed.

Figure 8 shows a TRUS with an attached biopsy guide that holds a needle. The needle extends into the plane of the TRUS image so that it is continuously visible therein.

While the method of performing biopsy has been described with specificity to manual biopsy needle insertion using a template, other types of biopsy needle insertion methods will occur to those of skill in the art. For example, insertion and/or alignment of the biopsy needle can be performed in a number of manners. In one embodiment, a robotic assembly is used to control the alignment and insertion of the biopsy needle. In another embodiment, a computer is used to control the needle guide in order to control the alignment of the biopsy needle, but still permits manual control of its insertion. In still another embodiment, via a robot or can be computer-controlled.

In a further embodiment, an end-firing US transducer can be coupled to a magnetic tracking device that provides position information to the computer. In this manner, 2D images with position and orientation measurements are simultaneously acquired using a free-hand magnetically tracked approach and are then reconstructed into 3D TRUS images in real-time. A free-hand magnetically or optically tracked scanning approach is used to allow the user to manipulate the transducer freely, and record the position and orientation of the transducer in space. The magnetic tracking approach is based on a small 6 degree-of-freedom magnetic field sensor (receiver) mounted on the TRUS transducer, and a transmitter is placed near the patient to produces a spatially varying magnetic field. The small sensor measures the three components of the local magnetic field strength, and these are used to calculate the TRUS transducer's position and orientation, which are then used in the 3D reconstruction algorithm.

In still yet another embodiment, markers can be attached to the TRUS transducer and a camera tracks movement of the markers in order to determine the position and orientation of the TRUS transducer.

The above-described embodiments are intended to be examples and alterations and modifications may be effected thereto, by those of skill in the art, without departing from the scope of the invention which is defined by the claims appended hereto.

**Claims**

1. An apparatus (1) for guiding a medical tool (40), comprising:

    a medical tool (40);
    at least one crank arm (2) comprising at least a portion of a first hinged coupling (10) for hinged coupling to a stabilizer (70);

at least one link arm (4) comprising at least a portion (20) of a second hinged coupling (16) for hinged coupling to the crank arm at a location spaced from the first hinged coupling;

a tool holder (6) for supporting a medical tool on the link arm at a location spaced from the first hinged coupling;

wherein the rotational axes of the first and second hinged couplings intersect to define a remote fulcrum (0);

wherein the apparatus comprises a shaft (32), for actuating longitudinal and rotational motion of the medical tool (40), **characterized in that** the medical tool (40) comprises a combination of an ultrasound transducer (46), a biopsy needle (44) and a needle guide (42) for allowing collection of ultrasound images for use in guiding operation of the needle (44).

2. The guide apparatus (1) of claim 1, further comprising:

a brake for inhibiting rotational motion of the first hinged coupling (10);
a brake for inhibiting rotational motion of the second hinged coupling (16); or
a first brake carried by the crank arm (2) and actuable to inhibit rotational motion of the first hinged coupling (10), and a second brake carried by the link arm (4) and actuable to inhibit rotational motion of the second hinged coupling (16).

3. The guide apparatus (1) of claim 1, further comprising:

a rotational encoder for sensing rotational motion of the first hinged coupling (10);
a rotational encoder for sensing rotational motion of the second hinged coupling (16); or
a first rotational encoder (60) carried by the crank arm (2) for sensing rotational motion of the first hinged coupling (10), and a second rotational encoder (62) carried by the link arm (4) for sensing rotational motion of the second hinged coupling (16).

4. The guide apparatus (1) of claim 1, further comprising:

a motor for controlling rotational motion of the first hinged coupling (10);
a motor for controlling rotational motion of the second hinged coupling (16); or
a first motor carried by the crank arm (2) actuable to control rotational motion of the first hinged coupling (10), and a second motor carried by the link arm (4) actuable to control rotational motion of the second hinged coupling (16).

5. The guide apparatus (1) of claim 1, further comprising:

a counterweight or spring balance for offsetting mass of the crank arm (2) and the link arm (4);
another counterweight or spring balance carried by the link arm (4) adjacent to the second hinged coupling (16); or
a counterweight (50) or spring balance carried by the crank arm (2) adjacent to the first hinged coupling (10), and another counterweight (52) or spring balance carried by the link arm (4) adjacent to the second hinged coupling (16).

6. The guide apparatus (1) of any one of claims 1 to 5, wherein the tool holder (6) axis passes through the remote fulcrum (0) thereby forming an open-loop spherical chain.

7. The guide apparatus (1) of any of claims 1 to 5 wherein the shaft (32) for actuating the medical tool (40) is coupled to a cylindrical joint of the tool holder.

8. The guide apparatus (1) of claim 1, wherein the shaft axis passes through the remote fulcrum (0) thereby forming an open-loop spherical chain.

9. The guide apparatus (1) of claim 1, further comprising:

a second crank arm coupled to the first hinged coupling (10); and
a second link arm coupled to the tool holder (6); and
a third hinged coupling for hinged coupling of the second crank arm and the second link arm at a location spaced from the first hinged coupling and the tool holder.

10. The guide apparatus (1) of claim 8, wherein the tool holder (6) comprises two rings each independently coupled to the link arms.

11. The guide apparatus (1) of claim 1 or 8, further comprising a medical tool (40).

12. The guide apparatus (1) of claim 10, further comprising a counterweight (52) for offsetting the mass of the medical tool (40).

13. The guide apparatus (1) of claim 10, wherein the axis of the medical tool (40) passes through the remote fulcrum (0).

14. The guide apparatus (1) of claim 1 or 8, wherein the shaft (32), is coupled to a differential gear train housed within the tool holder (6).

15. The guide apparatus (1) of claim 13, further comprising first and second rotational encoders housed within the tool holder (6), for measuring angular displacement of a base drum (121) and outer ring (122), respectively, of the differential gear train.

16. The guide apparatus (1) of any one of claims 1 to 14, wherein the crank arm (2) and the link arm (4) are each of an arcuate shape having a central angle of less than 90 degrees.

17. The guide apparatus (1) of claim 1, further comprising:

a second crank arm coupled to the first hinged coupling (10); and
a link arm assembly acting between each crank arm (2) and the tool holder (6), each link arm assembly comprising at least two hingedly coupled link arms.


**Patentansprüche**

1. Vorrichtung (1) zum Führen eines medizinischen Instruments (40), die Folgendes umfasst:

ein medizinisches Instrument (40),
mindestens einen Kurbelarm (2) mit zumindest einem Abschnitt einer ersten Gelenkverbindung (10) für eine Gelenkverbindung mit einem Stabilisator (70),
mindestens einen Verbindungsarm (4) mit zumindest einem Abschnitt (20) einer zweiten Gelenkverbindung (16) für eine Gelenkverbindung mit dem Kurbelarm an einer von der ersten Gelenkverbindung beabstandeten Stelle,
einen Instrumentenhalter (6) zum Halten eines medizinischen Instruments an dem Verbindungsarm an einer von der ersten Gelenkverbindung beabstandeten Stelle,
wobei sich die Drehachsen der ersten und der zweiten Gelenkverbindung so schneiden,
dass sie einen entfernt liegenden Drehpunkt (0) definieren,
wobei die Vorrichtung eine Welle (32) zum Bewirken einer Längs- und einer Drehbewegung des medizinischen Instruments (40) umfasst,
**dadurch gekennzeichnet, dass** das medizinische Instrument (40) eine Kombination aus einem Ultraschallwandler (46), einer Biopsienadel (44) und einer Nadelführung (42) umfasst, damit eine Erfassung von Ultraschallbildern zur Verwendung beim Führen der Operation der Nadel (44) möglich ist.

2. Führungsvorrichtung (1) nach Anspruch 1, die ferner Folgendes umfasst:

eine Bremse zum Unterbinden einer Drehbewegung der ersten Gelenkverbindung (10),
eine Bremse zum Unterbinden einer Drehbewegung der zweiten Gelenkverbindung (16)
oder
eine erste Bremse, die von dem Kurbelarm (2) getragen wird und dazu betätigbar ist,
eine Drehbewegung der ersten Gelenkverbindung (10) zu unterbinden, und eine zweite Bremse, die von dem Verbindungsarm (4) getragen wird und dazu betätigbar ist, eine Drehbewegung der zweiten Gelenkverbindung (16) zu unterbinden.

3. Führungsvorrichtung (1) nach Anspruch 1, die ferner Folgendes umfasst:

einen Drehgeber zum Erfassen einer Drehbewegung der ersten Gelenkverbindung (10),
einen Drehgeber zum Erfassen einer Drehbewegung der zweiten Gelenkverbindung (16)
oder
einen ersten Drehgeber (60) zum Erfassen einer Drehbewegung der ersten Gelenkverbindung (10), der von dem Kurbelarm (2) getragen wird, und einen zweiten Drehgeber (62) zum Erfassen einer Drehbewegung der zweiten Gelenkverbindung (16),
der von dem Verbindungsarm (4) getragen wird.

4. Führungsvorrichtung (1) nach Anspruch 1, die ferner Folgendes umfasst:

einen Motor zum Steuern einer Drehbewegung der ersten Gelenkverbindung (10),
einen Motor zum Steuern einer Drehbewegung der zweiten Gelenkverbindung (16) oder
einen ersten Motor, der von dem Kurbelarm (2) getragen wird und dazu betätigbar ist,
eine Drehbewegung der ersten Gelenkverbindung (10) zu steuern, und einen zweiten Motor, der von dem Verbindungsarm (4) getragen wird und dazu betätigbar ist, eine Drehbewegung der zweiten Gelenkverbindung (16) zu steuern.

5. Führungsvorrichtung (1) nach Anspruch 1, die ferner Folgendes umfasst:

ein Ausgleichsgewicht oder einen Federausgleich zum Ausgleichen der Masse des Kurbelarms (2) und des Verbindungsarms (4),
ein weiteres Ausgleichsgewicht oder einen weiteren Federausgleich, das/der von dem Verbindungsarm (4) getragen wird, neben der zweiten Gelenkverbindung (16) oder
ein Ausgleichsgewicht (50) oder einen Federausgleich, das/der von dem Kurbelarm (2) getragen wird, neben der ersten Gelenkverbindung (10) und ein weiteres Ausgleichsgewicht (52) oder ein weiterer Federausgleich, das/der von dem Verbindungsarm (4) getragen wird, neben der zweiten Gelenkverbindung (16).

6. Führungsvorrichtung (1) nach einem der Ansprüche 1 bis 5, wobei die Achse des Instrumentenhalters (6) durch den entfernt liegenden Drehpunkt (0) verläuft, wodurch eine offene, kugelförmige Kette entsteht.

7. Führungsvorrichtung (1) nach einem der Ansprüche 1 bis 5, wobei die Welle (32) zum Betätigen des medizinischen Instruments (40) mit einem zylinderförmigen Gelenk des Instrumentenhalters verbunden ist.

8. Führungsvorrichtung (1) nach Anspruch 1, wobei die Wellenachse durch den entfernt liegenden Drehpunkt (0) verläuft, wodurch eine offene, kugelförmige Kette entsteht.

9. Führungsvorrichtung (1) nach Anspruch 1, die ferner Folgendes umfasst:

einen zweiten Kurbelarm, der mit der ersten Gelenkverbindung (10) verbunden ist, und
einen zweiten Verbindungsarm, der mit dem Instrumentenhalter (6) verbunden ist, und
eine dritte Gelenkverbindung zur Gelenkverbindung des zweiten Kurbelarms und
einen zweiten Verbindungsarm an einer von der ersten Gelenkverbindung und dem Instrumentenhalter beabstandeten Stelle.

10. Führungsvorrichtung (1) nach Anspruch 8, wobei der Instrumentenhalter (6) zwei Ringe umfasst, die jeweils eigenständig mit den Verbindungsarmen verbunden sind.

11. Führungsvorrichtung (1) nach Anspruch 1 oder 8, die ferner ein medizinisches Instrument (40) umfasst.

12. Führungsvorrichtung (1) nach Anspruch 10, die ferner ein Ausgleichsgewicht (52) zum Ausgleichen der Masse des medizinischen Instruments (40) umfasst.

13. Führungsvorrichtung (1) nach Anspruch 10, wobei die Achse des medizinischen Instruments (40) durch den entfernt liegenden Drehpunkt (0) verläuft.

14. Führungsvorrichtung (1) nach Anspruch 1 oder 8, wobei die Welle (32) mit einem Differentialgetriebe verbunden ist, das in dem Instrumentenhalter (6) untergebracht ist.

**15.** Führungsvorrichtung (1) nach Anspruch 13, die ferner jeweils einen in dem Instrumentenhalter (6) untergebrachten ersten und zweiten Drehgeber zum Messen einer Winkelverschiebung eines Grundzylinders (121) beziehungsweise eines Außenrings (122) des Differentialgetriebes umfasst.

**16.** Führungsvorrichtung (1) nach einem der Ansprüche 1 bis 14, wobei der Kurbelarm (2) und der Verbindungsarm (4) jeweils eine Bogenform mit einem Mittelpunktswinkel von weniger als 90 Grad aufweisen.

**17.** Führungsvorrichtung (1) nach Anspruch 1, die ferner Folgendes umfasst:

einen zweiten Kurbelarm, der mit der ersten Gelenkverbindung (10) verbunden ist, und
eine Verbindungsarmbaugruppe, die zwischen jedem Kurbelarm (2) und dem Instrumentenhalter (6) wirkt, wobei jede Verbindungsarmbaugruppe mindestens zwei angelenkte Verbindungsarme umfasst.


**Revendications**

**1.** Appareil (1) pour guider un outil médical (40), comprenant :

un outil médical (40) ;
au moins un bras de manivelle (2) comprenant au moins une partie d'un premier couplage articulé (10) pour le couplage articulé à un stabilisateur (70) ;
au moins un bras de liaison (4) comprenant au moins une partie (20) d'un deuxième couplage articulé (16) pour le couplage articulé au bras de manivelle au niveau d'un emplacement espacé du premier couplage articulé ;
un support d'outil (6) pour supporter un outil médical sur le bras de liaison au niveau d'un emplacement espacé du premier couplage articulé ;
dans lequel les axes de rotation des premier et deuxième couplages articulés se coupent pour définir un pivot éloigné (0)
dans lequel l'appareil comprend un arbre (32), pour actionner le mouvement longitudinal et de rotation de l'outil médical (40), **caractérisé en ce que** l'outil médical (40) comprend une combinaison d'un transducteur à ultrasons (46), d'une aiguille à biopsie (44) et d'un guide pour aiguille (42) pour permettre la collecte d'images ultrasonores destinées à être utilisées dans l'opération de guidage de l'aiguille (44).

**2.** Appareil de guidage (1) selon la revendication 1, comprenant en outre :

un frein pour inhiber le mouvement de rotation du premier couplage articulé (10) ;
un frein pour inhiber le mouvement de rotation du deuxième couplage articulé (16) ; ou
un premier frein porté par le bras de manivelle (2) et pouvant être actionné pour inhiber le mouvement de rotation du premier couplage articulé (10), et un deuxième frein supporté par le bras de liaison (4) et pouvant être actionné pour inhiber le mouvement de rotation du deuxième couplage articulé (16).

**3.** Appareil de guidage (1) selon la revendication 1, comprenant en outre :

un codeur rotatif pour détecter le mouvement de rotation du premier couplage articulé (10) ;
un codeur rotatif pour détecter le mouvement de rotation du deuxième couplage articulé (16) ;
ou
un premier codeur rotatif (60) porté par le bras de manivelle (2) pour détecter le mouvement de rotation du premier couplage articulé (10), et un deuxième codeur rotatif (62) porté par le bras de liaison (4) pour détecter le mouvement de rotation du deuxième couplage articulé (16).

**4.** Appareil de guidage (1) selon la revendication 1, comprenant en outre :

un moteur pour commander le mouvement de rotation du premier couplage articulé (10) ;
un moteur pour commander le mouvement de rotation du deuxième couplage articulé (16) ;
ou
un premier moteur porté par le bras de manivelle (2) pouvant être actionné pour commander le mouvement de rotation du premier couplage articulé (10), et un deuxième moteur porté par le bras de liaison (4) pouvant être actionné pour commander le mouvement de rotation du deuxième couplage articulé (16).

**5.** Appareil de guidage (1) selon la revendication 1, comprenant en outre :

un contrepoids ou une balance à ressort pour compenser la masse du bras de manivelle (2) et du bras de liaison (4) ;
un autre contrepoids ou une autre balance à ressort porté par le bras de liaison (4) adjacent au deuxième couplage articulé (16) ; ou
un contrepoids (50) ou une autre balance à ressort porté par le bras de manivelle (2) adjacent au premier couplage articulé (10), et un autre contrepoids (52) ou une autre balance à ressort porté par le bras de liaison (4) adjacent au deuxième couplage articulé (16).

**6.** Appareil de guidage (1) selon l'une quelconque des revendications 1 à 5, dans lequel l'axe de support d'outil (6) passe à travers le pivot éloigné (0) formant ainsi une chaîne sphérique à boucle ouverte.

**7.** Appareil de guidage (1) selon l'une quelconque des revendications 1 à 5 dans lequel l'arbre (32) pour actionner l'outil médical (40) est couplé à un joint cylindrique du support d'outil.

**8.** Appareil de guidage (1) selon la revendication 1, dans lequel l'axe d'arbre passe à travers le pivot éloigné (0) formant ainsi une chaîne sphérique à boucle ouverte.

**9.** Appareil de guidage (1) selon la revendication 1, comprenant en outre :

un deuxième bras de manivelle couplé au premier couplage articulé (10) ; et
un deuxième bras de liaison couplé au support d'outil (6) ; et
un troisième couplage articulé pour le couplage articulé du deuxième bras de manivelle et du deuxième bras de liaison au niveau d'un emplacement espacé du premier couplage articulé et du support d'outil.

**10.** Appareil de guidage (1) selon la revendication 8, dans lequel le support d'outil (6) comprend deux bagues étant chacune couplées indépendamment aux bras de liaison.

**11.** Appareil de guidage (1) selon la revendication 1 ou 8, comprenant en outre un outil médical (40).

**12.** Appareil de guidage (1) selon la revendication 10, comprenant en outre un contrepoids (52) pour compenser la masse de l'outil médical (40).

**13.** Appareil de guidage (1) selon la revendication 10, dans lequel l'axe de l'outil médical (40) passe à travers le pivot éloigné (0).

**14.** Appareil de guidage (1) selon la revendication 1 ou 8, dans lequel l'arbre (32) est couplé à un train d'engrenage différentiel logé au sein du support d'outil (6).

**15.** Appareil de guidage (1) selon la revendication 13, comprenant en outre des premier et deuxième codeurs rotatifs logés au sein du support d'outil (6), pour mesurer le déplacement angulaire d'un tambour de base (121) et d'une bague externe (122), respectivement, du train d'engrenage différentiel.

**16.** Appareil de guidage (1) selon l'une quelconque des revendications 1 à 14, dans lequel le bras de manivelle (2) et le bras de liaison (4) ont chacun la forme d'un arc ayant un angle central de moins de 90 degrés.

**17.** Appareil de guidage (1) selon la revendication 1, comprenant en outre :

un deuxième bras de manivelle couplé au premier couplage articulé (10) ; et
un assemblage de bras de liaison agissant entre chaque bras de liaison (2) et le support d'outil (6), chaque assemblage de bras de liaison comprenant au moins deux bras de liaison couplés de manière articulée.

FIG. 1

EP 2 034 921 B1

FIG. 2

FIG. 1(a)

FIG. 1(b)

FIG. 1(c)

EP 2 034 921 B1

360°

FIG. 4(a)

90°

FIG. 4(b)

FIG. 5

FIG. 6(c)

FIG. 6(b)

FIG. 6(a)

FIG. 7

FIG. 7(a)

FIG. 7(b)

FIG. 7(c)

EP 2 034 921 B1

FIG. 8(a)

FIG. 8(b)

FIG. 8(c)

EP 2 034 921 B1

FIG. 9(a)

FIG. 9(b)

FIG. 9(c)

24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5397323 A
- US 5515478 A
- US 5630431 A
- US 5817084 A
- US 5907664 A
- US 6047610 A
- US 6246200 B
- US 7021173 B
- US 5257998 A
- US 4723544 A
- US 4669483 A